# EUROPEAN PATENT SPECIFICATION

(11) **EP 0 723 453 B1**
(45) Date of publication and mention of the grant of the patent: **15.03.2000**
(21) Application number: 94926472.5
(22) Date of filing: 02.08.1994
(51) Int. Cl.: A61K 38/30, C07K 7/00, C07K 14/00

(54) **METHOD OF TREATING REPRODUCTIVE DISORDERS**
METHODE ZUR BEHANDLUNG VON REPRODUKTIONSERKRANKUNGEN
PROCEDE DE TRAITEMENT DE DYSFONCTIONNEMENTS DE LA REPRODUCTION

(30) Priority: 03.08.1993 US 101255
(43) Date of publication of application: 31.07.1996
(73) Proprietor: CELTRIX PHARMACEUTICALS, INC., Santa Clara, CA 95052-8203 (US)
(72) Inventor: MAACK, Christopher A., El Cerrito, CA 94530 (US)
(74) Representative: Goldin, Douglas Michael
(86) International application number: US9408627
(87) International publication number: WO9503817

(56) References cited:
- WO-A-89/08667
- DATABASE CHEMICAL ABSTRACTS FILE SERVER STN KARLSRUHE ABSTRACT 118:184273, SOEDER ET AL: "REGULATION OF GERM CELL PROLIFERATION BY GROWTH FACTORS" XP002019600
- DATABASE MEDLINE FILE SERVER STN KARLSRUHE ABSTRACT 92091462, VOLPE ET AL: "CLINICAL USE OF GROWTH HORMONE-RELEASING FACTOR FOR INDUCTION OF SUPEROVULATION" XP002019601
- HUMAN REPRODUCTION, Volume 4, Number 1, issued 1989, GEISTHOEVEL et al., "Immunoreactive Insulin-Like Growth Factor I in Human Follicular Fluid", pages 35-38.
- JOURNAL OF REPRODUCTION AND FERTILITY, Volume 96, Number 2, issued November 1992, JIMENA et al., "Insulin and Insulin-Like Growth Factor I in Follicular Fluid after Induction of Ovulation in Women Undergoing in Vitro Fertilization", pages 641-647.
- HUMAN REPRODUCTION, Volume 7, Number 10, issued 1992, VOLPE et al., "Effects of Growth Hormone Administration in Addition to Gonadotrophins in Normally Ovulating Women and Polycystic Ovary Syndrome (PCO) Patients", pages 1347-1352.
- ANNALS OF THE NEW YORK ACADEMY OF SCIENCES, Volume 626, issued 1991, LUNENFELD et al., "Modern Aspects of Ovulation Induction", pages 207-216.
- FERTILITY AND STERILITY, Volume 57, Number 5, issued May 1992, SHOHAM et al., "Cotreatment with Growth Hormone for Induction of Spermatogenesis in Patients with Hypogonadotropic Hypogonadism", pages 1044-1051.
- HUMAN RERPODUCTION, Volume 5, (Supplement), issued 1990, TOROK et al., "Insulin-Like Growth Factors and Their Binding Proteins in Serum and Follicular Fluid of Patients Undergoing in Vitro Fertilization", pages 58-59.

## Description

### Field of the Invention

This process relates generally to the field of medical therapy and particularly to medicaments for the treatment of reproductive disorders by administering a therapeutic composition containing a complex of an insulin-like growth factor (IGF) and an insulin-like growth factor binding protein (IGFBP).

### Background Art

Growth factors are polypeptides which stimulate a wide variety of biological responses (eg. DNA synthesis, cell division, expression of specific genes, etc.) in a defined population of target cells. A variety of growth factors have been identified including transforming growth factor-β1 (TGF-β1), TGF-β2, TGF-β3, epidermal growth factor (EGF), platelet-derived growth factor (PDGF), fibroblast growth factor (FGF), insulin-like growth factor-I (IGF-I), and IGF-II.

IGF-I and IGF-II are related in sequence and structure, with each polypeptide having a molecular weight of approximately 7500 daltons. IGF-I mediates the effects of growth hormone and thus is the primary mediator of growth after birth. IGF-I has also been implicated in the actions of various other growth factors, since treatment of cells with such growth factors leads to increased production of IGF-I. In contrast, IGF-II is believed to have a major role in fetal growth. Both IGF-I and IGF-II have insulin-like activities (hence the name) and are mitogenic (stimulate cell division) for the cells in reproductive tissue, muscle, skeletal tissue and a wide variety of other tissues.

Unlike most growth factors, the IGFs are present in substantial quantity in the circulation, but only a very small fraction of this IGF is found in the free form in the circulation or in other body fluids. Most circulating IGF is bound to an IGF-binding protein called IGFBP-3. IGF-I may be measured in blood serum in which it is found at abnormally low levels in abnormal growth-related conditions, eg, pituitary gigantism, acromegaly, dwarfism, various growth hormone deficiencies, etc. Although IGF-I is produced in many tissues, most circulating IGF-I is believed to be synthesized in the liver.

Almost all IGF circulates in a non-covalently associated ternary complex composed of IGF-I or IGF-II, IGFBP-3, and a larger protein termed the acid labile subunit (ALS). This complex is composed of equimolar amounts of each of the three components. The ALS has no direct IGF binding activity and appears to bind only a preformed IGF/IGFBP-3 complex. The ternary complex of IGF + IGFBP-3 + ALS has a molecular weight of approximately 150,000 daltons. This ternary complex is alleged to function in the circulation "as a reservoir and a buffer for IGF-I and IGF-II preventing rapid changes of free IGF." See, Blum, W.F., et al., "Plasma IGFBP-3 Levels as Clinical Indicators", In Modern Concepts in Insulin-Like Growth Factors, (E. M. Spencer, ed., Elsevier, New York) pages 381-393, 1991.

Nearly all of the IGF-I or IGF-II and IGFBP-3 in the circulation are in complexes, so very little free IGF or IGFBP-3 is detectable. Moreover, a high level of free IGF in plasma is undesirable. It would lead to serious hypoglycemia because IGF has insulin-like effects on circulating glucose levels. In contrast to the IGFs and IGFBP-3, there is a substantial pool of free ALS in plasma which assures that IGF/IGFBP-3 complex entering the circulation immediately forms the ternary complex.

IGFBP-3 is the most abundant IGF-binding protein in the circulation, but at least five other distinct IGF-binding proteins (IGFBPs) have been identified in various tissues and body fluids. Although these proteins bind IGFs, they each originate from separate genes and have distinct amino acid sequences. Thus, the binding proteins are not merely analogs of a common precursor. Unlike IGFBP-3, the other IGFBPs in the circulation are not saturated with IGFs. None of the IGF binding proteins other than IGFBP-3 can form the 150 kd circulating ternary complex.

IGF-I and IGFBP-3 may be purified from natural sources or produced from recombinant sources. For instance, IGF-I has been purified from human serum for a number of years. See, Rinderknecht, E.W., et al., Proc Natl Acad Sci (USA) 73, 2365-2369 (1976). Recombinant IGF-I processes are shown in EPA 0,128,733, published in December of 1984. IGFBP-3 may be purified from natural sources using processes such as those shown in Baxter et al., Biochem. Biophys. Res. Comm. 139, 1256-1261 (1986). IGFBP-3 may be synthetically produced from recombinant sources as discussed in Sommer, A. S., et. al., In Modern Concepts of Insulin-Like Growth Factors (E. M. Spencer, ed., Elsevier, New York), pages 715-728 (1991). This recombinant IGFBP-3 binds IGF-I in a 1:1 molar ratio. The topical administration of the IGF-I/IGFBP-3 complex to rat and pig wounds was significantly more effective than IGF-I alone. Sommer et al., ibid. Subcutaneous administration of the complex to hypophysectomized rats "substantially prevents the hypoglycemic effects of IGF-I" administered alone. Sommer et al., ibid.

U.S. Patent No. 5,037,805 issued to Ling discloses a method for using a follicle-stimulating hormone inhibiting protein for treating endometriosis and for use as a contraceptive. This protein is identical to IGFBP-3. There is no suggestion to use this protein in conjunction with IGF.

Patent Cooperation Treaty Publication No. WO 91/14704, published on October 3, 1991, and applied for by Applied Research Systems ARS Holding N.V. discloses a method for using estrogen to increase endogenous IGFBP-1 as a treatment for polycystic ovarian disease (PCOD). The theory was advanced that because PCOD is associated with higher levels of free IGF-1 and lower levels of IGFBP-1, administering estrogen to increase IGFBP-1 would decrease levels of free IGF and reduce follicular overstimulation and overproduction of androgens. The use of exogenous IGFBP-1 was not proposed.

All of the important elements of the IGF system are found in the female reproductive organs of humans and other mammals, including IGF-I and -II and the IGF binding proteins and receptors. IGF-I and IGF-II have been implicated in the growth and differentiated function of several of the tissues of these organs. The presence of IGF-I and IGF-II in rat, pig and human ovarian follicular fluid has been well documented. The presence of IGF-I and IGF-II mRNAs in ovarian granulosa and theca-interstitial cells suggests that at least some of the IGF found in follicular fluid arises from local production in the surrounding cells. In fact, of all of the tissues in the rat, the ovary has the third highest level of IGF-I mRNA. In humans, IGF-I synthesis may be restricted to the theca-interstitial cells, while IGF-II production appears to occur only in granulosa cells. The expression of the IGF-I genes in the ovary seems to be under complex hormonal control. *In vitro* studies indicate that growth hormone, gonadotropins, estrogen, and epidermal growth factor stimulate production of murine and porcine granulosa cell IGF-I; combination treatment often results in a synergistic stimulation of IGF-I gene expression. There are some inter-species differences observed in the hormonal control of IGF-I production.

Type I IGF receptors, which act to transduce the mitogenic and differentiation signals provided by the IGFs, have also been demonstrated in mouse, pig and human ovarian granulosa and theca-interstitial cells. The gonadotropins, follicle stimulating hormone (FSH) and luteinizing hormone (LH), are pivotal hormones in controlling the progression of differentiation events that lead to a mature fertilizable ovum. FSH and LH have been shown to stimulate an increase in the Type I IGF receptors in granulosa cells. Substantial quantities of the Type II IGF receptor are found in the ovary but the function of this receptor is obscure.

The third element of the IGF system, the IGF binding proteins, are also synthesized in the ovary and are found in ovarian follicular fluid. IGFBP-2, -3, - 4, -5 and -6 have been identified in porcine follicular fluid, while IGFBP-1, -2, -3 and -4 are found in fluid from luteinizing human follicles. In the rat, mRNAs for IGFBP-2, -3, -4 and -5 are detectable in the adult ovary but IGFBP-1 is not. However, mRNAs for IGFBP-1, -2, - 3, -4 and -5 are present in particular cell types in the human ovary. IGFBP-2, -4, and -5 mRNAs were found primarily in granulosa cells from atretic (i.e. degenerating) follicles. IGFBP-1 and IGFBP-3 mRNAs were found in granulosa cells of dominant (i.e. mature) follicles and IGFBP-3 mRNA, additionally in theca-interstitial cells.

As with IGF-I, the hormonal regulation of the IGFBPs is quite complex. In rats, ovarian IGFBP-2 and -3 mRNA was reduced after hypophysectomy, but subsequent treatment with diethylstilbestrol (DES) led to an increase in IGFBP-2 mRNA and a decrease in IGFBP-3 mRNA. Growth hormone treatment increased IGFBP-3 mRNA in hypophysectomized rats, an effect which was antagonized by DES or FSH. The response of the rat ovary and of cultured rat ovarian granulosa cells to FSH was also complex, both *in vivo* and *in vitro*. Low doses of FSH led to an increase in the secretion of total IGFBPs, while higher doses depressed IGFBP secretion.

As with many other tissues, IGF treatment of the various cell types of the female reproductive system leads to both mitogenesis and the expression of tissue-specific differentiated functions. Presumably acting through the Type I IGF receptors, both IGF-I and IGF-II have been shown to stimulate granulosa cell DNA synthesis and proliferation in rats and pigs. Growth factors and hormones (such as epidermal growth factor, platelet derived growth factor, FSH and relaxin) can act synergistically with IGF-I to augment this mitogenic effect.

The IGFs, either alone or with FSH or LH, can stimulate sex steroid production in certain reproductive cells, such as granulosa and theca-interstitial cells. For example, IGF-I alone stimulates aromatase activity, as well as estrogen and progesterone synthesis, in granulosa cells in a variety of species. In synergy with FSH or LH, IGF-I treatment of granulosa cells 1) promotes a higher rate of estrogen and progesterone production than occurs with either hormone alone and 2) stimulates proteoglycan and LH receptor production. Similarly, IGF-I acts synergistically with LH to increase androgen production in cultured theca-interstitial cells. In summary, many gonadotropin and sex steroid effects on ovarian function may be facilitated or potentiated by endogenous or exogenous IGFs in developing follicles.

IGF and IGFBP actions have been studied in cultured rat granulosa cells. Adding excess IGFBP-1, -2, or -3 to these cells blocks the synergistic effects of IGF-I and FSH treatment on mitogenesis and steroid production. This presumably occurs by competition for IGFs between the free IGFBPs and the IGF Type I receptor.

Polycystic ovarian disease (PCOD) has been extensively studied. PCOD is a type of infertility characterized by hyperandrogenism which prevents small ovarian follicles from becoming dominant and maturing to the point of release and fertilization. Several studies have analyzed IGF and IGFBP levels in ovarian follicles at different stages of maturation in normally ovulating and PCOD patients. Follicular fluids contain IGF-I and IGFBP-2, -3, and -4, as well as a 29 kd IGFBP. IGFBP-3 is the predominant IGFBP in follicular fluid. There were no differences in IGF-I or IGFBP-3 levels in the fluid from atretic (degenerating) follicles obtained from normal or PCOD patients compared to fluid from healthy follicles from normal patients. However, the levels of IGFBP-2, -4, and 29 kd IGFBP were elevated in fluid from atretic follicles obtained from normal or PCOD patients compared to fluid from healthy follicles from normal patients. The total IGF binding capacity in atretic follicles was elevated compared to healthy follicles. Similar results were seen in fluid obtained from healthy and atretic follicles obtained from sheep. Since atretic follicular fluid is seriously deficient in estrogen and IGF-I stimulates production of estrogen by granulosa cells, it has been proposed that the abundant IGF-I in atretic follicular fluid is inactive because it binds to the increased IGFBP-2, -4, and 29 kd IGFBP. This apparently occurs without any change in IGFBP-3, the predominant IGFBP in follicular fluid.

In anovulatory women undergoing treatment to induce ovulation, growth hormone may sensitize ovarian tissues to the effects of gonadotropins. The standard therapy for ovulation induction is treatment with a gonadotropin releasing hormone (GnRH) agonist to suppress pituitary function, followed by repeated treatments with gonadotropins (LH, FSH and human chorionic gonadotropin (hCG)). The dose and length of gonadotropin treatment for each patient is guided by serum steroid hormone levels and follicular maturation as assessed by ultrasound. Several studies have used growth hormone in conjunction with standard gonadotropin therapy for ovulation induction. In a number of placebo-controlled studies of anovulatory patients who had previously undergone standard gonadotropin therapy, treatment with growth hormone reduced the daily dose of gonadotropin and shortened the course of treatment (resulting in a smaller cumulative gonadotropin dose) required to stimulate normal ovulation compared to that required by patients receiving placebo plus gonadotropins. This effect was accompanied by an increase in the level of IGF-I in serum and follicular fluid from growth hormone-treated patients compared to placebo-treated patients.

Women undergoing induction of superovulation for *in vitro* fertilization/embryo transfer (IVF/ET) have benefitted from combined growth hormone and gonadotropin therapy, especially in cases where their response to gonadotropin therapy alone has been suboptimal. For these patients, the clinical endpoint is the induction of multiple mature oocytes which can be successfully fertilized *in vitro*. As discussed above, several studies have shown that growth hormone with standard gonadotropin therapy significantly reduced the amount of gonadotropin required to induce superovulation in these "poor responders". Furthermore, co-administration of growth hormone and gonadotropins increased the number of mature oocytes that could be isolated from "poor responders", the growth rate of the harvested oocytes, and the success rate of oocyte fertilization *in vitro* and of viable pregnancies after embryo transfer. As described above, adding growth hormone to the standard therapeutic regimen significantly increased serum and follicular fluid IGF-I levels. Moreover, significantly higher IGF-I levels were found in follicular fluid from mature follicles that fertilized and cleaved than in follicular fluid from mature follicles that did not fertilize.

In contrast, the results of studies in normally ovulatory women undergoing superovulation therapy for IVF/ET have been mixed. Some studies have reported a reduced need for gonadotropins when they were co-administered with growth hormone, and other studies have reported no such reduction.

Growth hormone therapy has the disadvantage of causing hyperglycemia, hyperinsulinemia, sodium retention, edema, and growth hormone insensitivity when used for prolonged periods.

Although androgens, LH and FSH play major roles in the development and functioning of the male reproductive system, the importance of the growth hormone/IGF axis in male fertility is demonstrated by the striking delay in puberty and resistance to human chorionic gonadotropin (hCG) therapy experienced by boys with growth hormone resistance.

As with the female reproductive system, all three elements of the IGF system are present in the male reproductive tract. IGF-I is synthesized by both major cell types of the testis (Leydig and Sertoli cells). This synthesis appears to be primarily under the control of the gonadotropins rather than growth hormone. Similarly, the Type I IGF receptor is also present in both cell types, as well as in early spermatids and secondary spermatocytes. The IGF type I receptor is up-regulated by treatment with hCG. Finally, IGFBPs (predominantly IGFBP-2 and -3) are produced in several testicular cell types. In Sertoli cells IGFBP-2 and -3 expression can be regulated by IGF-I and FSH. IGF-I and IGFBP-2 and -3 are also produced in cultured prostatic cells and are found in seminal plasma.

IGF-I affects function of a number of testicular cells. In culture, IGF-I is mitogenic for Sertoli cells and stimulates plasminogen activator synthesis. IGF-I treatment of Leydig cells leads to increased hCG receptors and to synergy with hCG in stimulating androgen production.

The effects of IGF-I are not restricted to isolated cultured cells. In growth hormone deficient dwarf mice, IGF-I treatment increases testicular weight, LH receptor numbers, and the steroidogenic response to hCG. IGF-I is important in spermatogenesis: IGF-I treatment of sections of seminiferous tubules in organ culture stimulated premitotic DNA synthesis in the germ cells.

Growth hormone also has been used in male sterility. Four men with hypogonadotropic hypogonadism who had not responded to gonadotropins alone were given combined growth hormone and gonadotropin therapy. All four had an increase in serum IGF-I levels, three rapidly increased testosterone secretion, two produced adequate amounts of sperm, and one patient impregnated his wife. (Shoham et al., Fertility & Sterility 57:1044, 1992)

IGF given alone can cause hypoglycemia, cardiac arrhythmias and suppress growth hormone, insulin and ALS production.

As indicated above, many cases of female or male infertility caused by failures of gamete maturation are refractory to standard gonadotropin therapy. Such cases include, but are not limited to, polycystic ovarian disease patients and "poor responders" to gonadotropin-induced superovulation among females, and hypogonadotropic hypogonadism among males and require different or additional therapy.

### Disclosure of the Invention

In accordance with one embodiment of the present invention, the preparation of a medicament for treating a subject for a reproductive disorder associated with failure of gamete maturation is claimed, wherein the subject is administered standard gonadotropin therapy in conjunction with a complex comprising an insulin-like growth factor (IGF) and insulin-like growth factor binding protein-3 (IGFBP-3) in an amount sufficient to alleviate the reproductive disorder.

In accordance with another embodiment of the present invention, the IGF used in the complex is provided as IGF-I. In a further embodiment, IGF and IGFBP are present in equimolar amounts. In still another embodiment, both IGF and IGFBP-3 are human proteins obtained from recombinant sources.

In accordance with another embodiment of the present invention, the complex of IGF and IGFBP-3 is administered parenterally. In a further embodiment, the complex is administered by subcutaneous injection.

In another embodiment, the subject to whom the complex is administered is a mammal.

In yet another embodiment, the medicament provides for administration of the IGF/IGFBP-3 complex in an amount sufficient to increase serum estrogen in females and serum androgen in males and thus improve fertility. In a further embodiment, the amount of IGF/IGFBP-3 complex administered is about 0.01 to 10 mg/kg/day.

In still other embodiments, the medicament provides a treatment for polycystic ovarian disease, anovulation and male infertility.

While not wishing to be bound by any particular theory, the Inventors propose that the administered complex of IGF and IGFBP-3 results in the gradual release of free IGF in moderately elevated levels. This can occur either before or after the circulating IGF/IGFBP-3 complex is taken up into the testes or ovaries. The added IGF in the ovaries or testes sensitizes these tissues to the actions of gonadotropins, stimulates sex steroid production, stimulates germ cell division, and improves maturation and production of fertile mature oocytes and sperm.

### Modes For Carrying Out the Invention

### Definitions:

As used herein, "reproductive disorders" are defined as conditions that lead to reduced fertility due to the partial or total failure to produce fully mature oocytes or sperm which are capable of combining to produce a fertilized ovum. Such conditions include, but are not limited to, polycystic ovary disease (PCOD), failure to respond to gonadotropin therapy to induce normal ovulation, failure to respond to gonadotropin therapy to induce superovulation prior to *in vitro* fertilization/embryo transfer, anovulation, hypogonadotropic hypogonadism, and male infertility.

"Standard gonadotropin therapy" is defined as current clinically accepted standard treatments of infertile patients with a combination of gonadotropins and releasing factors. Such gonadotropins and releasing factors include, but are not limited to, follicle stimulating hormone (FSH), luteinizing hormone (LH), human menopausal gonadotropin (hMG), human chorionic gonadotropin (hCG) and gonadotropin releasing hormone (GnRH) agonists.

"Subjects" are defined as humans and mammalian farm animals, sport animals and pets. Farm animals include, but are not limited to, cows, hogs and sheep. Sport animals include, but are not limited to, dogs and horses. The category pets includes, but is not limited to, cats and dogs.

"Insulin-like growth factor (IGF)" comprises a family of factors, including, but not limited to, IGF-I and IGF-II. IGF is a polypeptide having a molecular weight of about 7500 daltons. IGF may be obtained from natural sources or prepared by recombinant means.

"Insulin-like growth factor binding proteins (IGFBP)" comprises a family of binding proteins, including but not limited to IGFBP-1, IGFBP-2, IGFBP-3, IGFBP-4, IGFBP-5 and IGFBP-6. IGFBP may be obtained from natural sources or prepared by recombinant means. At least one form of IGFBP (for example, IGFBP-3) complexes with IGF and with a third molecule known as ALS.

A "therapeutic composition" as used herein is defined as comprising IGF complexed with its binding protein IGFBP-3. The therapeutic composition also contains other substances such as water, minerals and carriers such as proteins.

"Alleviation of the condition" is said to occur when the subject to whom the IGF/IGFBP-3 complex is administered produces mature gametes capable of combining with a competent oocyte or sperm to produce a viable fertilized ovum. Alleviation has occurred when a male with a reproductive disorder produces normal quantities of normal, active sperm, or at least enough normal, active sperm to impregnate a competent oocyte (one capable of fertilization) . Alleviation has occurred when a female with a reproductive disorder ovulates and produces normal, competent oocyte(s) . There are other useful indicators of "alleviation," including, but not limited to, normal levels of estrogens or androgens.

### Description of the Invention

The medicament of the present invention contemplates treating and alleviating infertility caused by defective gamete maturation in males and females by administering a complex of IGF and IGFBP-3 in conjunction with standard gonadotropin therapy.

Nearly all IGF-I or IGF-II complexes with IGFBP-3. IGF/IGFBP-3 normally circulates in the form of a complex in humans and other mammals. This complex associates with a third protein (ALS), which is present in excess over the molar concentration of IGF and IGFBP-3. Therefore, ALS is found both associated with the IGF/IGFBP-3 complex and in the free form. The resultant ternary complex has a size of about 150 kd.

Administration of IGF and IGFBP-3, either from natural or recombinant sources, as a preformed complex results in the formation of the ternary complex with the excess ALS. This type of treatment produces a prolonged increase in the level of circulating IGF, which is gradually released from the ternary complex. This mode of administration avoids the detrimental side effects associated with administration of free IGF-I, e.g., hypoglycemia, suppression of growth hormone and ALS production, and sudden increase in free endogenous IGF-II released from normally circulating IGF-II/IGFBP-3 complexes by equilibrium replacement with exogenous free IGF-I in complexes with IGFBP-3.

The formulation, method of administration and dosage will depend upon the disorder to be treated and the medical history of the patient. These factors are readily determined in the course of therapy. Suitable patients with reproductive disorders can be identified by medical history, physical findings and laboratory tests. The medical history may reveal such facts as delayed puberty, female androgynism, irregular menstrual cycles, and repeated failure to conceive. The female patient may have polycystic ovarian disease, reduced response of immature ovarian follicles to standard gonadotropin therapy and anovulation; whereas the male patient may have a low sperm count. Indicative laboratory results include low serum growth hormone or IGF levels or reduced levels of serum estrogen (in females) or androgen (in males) in response to standard gonadotropin therapy.

In accordance with the present invention, the formulation comprises a complex of IGF and IGFBP-3. Preferably, the IGF is IGF-I, although IGF-II also is useful. Because IGF and IGFBP-3 naturally complex in a 1:1 molar ratio, a composition of equimolar amounts of IGF and IGFBP-3 is preferred. Nevertheless, the product can be formulated with IGF:IGFBP-3 molar ratios ranging from about 0.5 to 1.5. More preferably, the molar ratio is about 0.9 to 1.3; and most preferably, the product is formulated with approximately a 1:1 molar ratio.

In accordance with the present invention, the IGF and IGFBP-3 are human proteins obtained from natural or recombinant sources. Most preferably, IGF and IGFBP-3 are human IGF-I and IGFBP-3 made by recombinant means and designated rhIGF-I and rhIGFBP-3, respectively. rhIGFBP-3 may be in glycosylated or non-glycosylated form. *E. coli* is a preferred source of non-glycosylated IGFBP-3. Glycosylated IGFBP-3 is preferably obtained from Chinese hamster ovary (CHO) cells.

The medicament of the present invention provides for formulating the complex in modes which are readily apparent to those skilled in the art. Preferably, the IGF and IGFBP-3 are complexed prior to administration to the treated subject. Preferably, the complex is formed by mixing approximately equimolar amounts of IGF-I and IGFBP-3 dissolved in physiologically compatible carriers such as normal saline solution or phosphate buffer saline solution. Most preferably, a concentrated solution of rhIGF-I and a concentrated solution of rhIGFBP-3 are mixed together for a sufficient time to form an equimolar complex.

Depending on the mode of administration, compositions of the complex may be in the form of solid, semi-solid or liquid dosage preparations, such as for example, tablets, pills, powders, capsules, liquids, suspensions or the like. Physiologically compatible carriers include intravenous solutions, such as normal saline, serum albumin, 5% dextrose, plasma preparations, and other protein-containing solutions. The preferred carrier for parenteral administration of the complex is a sterile, isotonic aqueous solution, such as normal saline or 5% dextrose. Alternatively, a solution of the complex may be placed into an implant, such as an osmotic pump, for the slow release of the complex over an extended period of time. Alternatively, the complex may be provided in sustained release carrier formulations such as semi-permeable polymer carriers in the form of suppositories or microcapsules. See, for instance, U.S. Patent No. 3,773,919 for Microcapsular Sustained Release Matrices Including Polylactides; Sidmon et al., Biopolymers 22 (1): 547-556 (1983) for copolymers of L-glutamic acid and γ-ethyl-L-glutamate; Langer et al., J Biomed Res 15: 167-277 (1981) for poly(2-hydroxyethylmethacrylate) or the like.

The mode of administration delivers the complex to the subject in a safe, physiologically effective manner. The complex may be given by intranasal, subcutaneous, intravenous, intramuscular, intraperitoneal, or other conventional routes of administration. Preferably, the complex is injected subcutaneously, intravenously or intramuscularly. Most preferably, the complex is administered by subcutaneous injection. By subcutaneous injection, the complex appears not to be toxic or mitogenic at the injection site. Lack of mitogenic effect at the injection site is a distinct improvement over scarring following the injection of IGF alone.

The IGF/IGFBP complex is administered in conjunction with standard gonadotropin therapy, including gonadotropins and releasing factors. Such gonadotropins and releasing factors include, but are not limited to, follicle stimulating hormone (FSH), luteinizing hormone (LH), human menopausal gonadotropin (hMG), human chorionic gonadotropin (hCG) and gonadotropin releasing hormone (GnRH) agonists. FSH is available as Metrodin® urofollitropin (Serono Laboratories, Randolph, MA). hMG is obtained from the urine of post-menopausal women and includes equal activities of FSH and LH. hMG is available as PERGONAL® menotropins (Serono Laboratories) and as HUMEGON® menotropins (Organon Laboratories, Cambridge, U.K.). hCG is commercially available as PROFASI® HCG for Injection (Serono Laboratories), as well as under the brand names, CHORIGON® (Ikafarm, Ramat-Gan, Israel) and PREGNYL® (Organon, West Orange, NJ). GnRH agonists include LUPRON® leuprolide acetate (TAP Pharmaceuticals, Deerfield, IL) and DECAPEPTYL® triptorelin (Wyeth-Ayerst Laboratories, Philadelphia, PA), SUPREFACT buserelin acetate (Hoechst, Hounslow, U.K.). Prescribing information is available in the current edition of the P 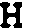 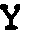 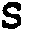 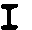 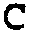 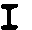 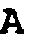 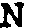 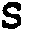 ' D 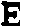 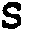 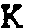 R 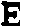 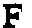 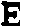 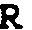 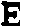 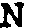 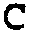 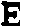 and in the clinical literature.

For female subjects, this standard therapy typically consists of ovarian suppression with a gonadotropin releasing hormone agonist, followed by induction of oocyte maturation and release with gonadotropin treatments. For example, the ovaries are suppressed by daily subcutaneous injections of GnRH agonist for up to 14 days starting in the early phase of the reproductive cycle. For example, 200-500 µg/d of SUPREFACT, 100-500 µg/d DECAPEPTYL or 500-1,000 µg/d LUPRON can be administered. Ovarian suppression is evaluated by measuring the serum estradiol level at the end of this initial period. If the level is below about 30-50 pg/ml, ovarian suppression has occurred and GnRH agonist treatments continue as gonadotropin treatments are begun. If the serum estradiol is above this level, GnRH treatment continues until ovarian suppression is achieved and again continues during gonadotropin treatment. Oocyte maturation is promoted by daily intramuscular injections of human menopausal gonadotropin (e.g. HUMEGON, PERGONAL, NEOPERGONAL, Serono, Levallois, France), a mixture of LH and FSH usually containing 150-225 IU of each hormone per ampule. Subjects are often give 2-3 ampules/day at first, with the dose adjusted to ensure a steady rise in serum estradiol in treated patients. Injections are given daily for at least 5-6 days and continue until the oocytes are mature (serum estradiol above 400-500 pg/ml and 1-3 follicles greater than 14-18 mm diameter). Finally, ovulation is induced by a single intramuscular injection of 5,000-10,000 IU of human chorionic gonadotropin (e.g. PROFASI, PREGNYL or CHORIGON). Therapy with rhIGF-I/IGFBP-3 complex is combined with standard gonadotropin therapy as discussed below.

Standard gonadotropin therapy for male subjects with failures of spermatogenesis includes, for example, either pulsatile dosing with luteinizing hormone releasing hormone (LH-RH) or gonadotropin therapy in order to raise serum testosterone levels and induce sperm maturation. LH-RH (e.g. FERTIRAL brand, available from Hoechst, Hounslow, U.K.) can, for example, be given as 15 µg subcutaneous pulses every 90 minutes with a self-driven pump. For subjects who do not respond to LH-RH therapy, hMG can be administered three times per week and hCG twice per week for 3-6 months by intramuscular injection. In other embodiments, these regimens are combined with administration of the rhIGF-I/IGFBP-3 complex.

The appropriate dose of IGF/IGFBP complex can be readily determined by those skilled in the art, based on the usual patient symptoms and laboratory values discussed above. In addition, patient estrogen and androgen levels may be helpful. Typically, the peak serum estradiol levels in hyperstimulated female subjects should rise to approximately 1,000 - 3,000 pg/ml, or at least 200 pg/ml/mature follicle. For treated male subjects, serum testosterone levels should rise to approximately 300 - 1,000 ng/dl; and the sperm count should increase toward the normal 20-250 million/ml, while motility should increase, preferably toward 50%. Preferably, when the complex is administered to humans daily, the dosage of complex is about 0.01 to 10 mg of IGF-I or IGF-II/kg of body weight/day, complexed to an approximately equimolar amount of IGFBP-3. More preferably, the daily dosage of the complex for humans is about 0.05 to 7.5 mg IGF/kg/day, complexed to an equimolar amount of IGFBP-3. Most preferably, the daily dosage of the complex for humans is about 0.1 to 5 mg IGF/kg/day, complexed to an equimolar amount of IGFBP-3. If daily dosages in excess of about 0.5 mg IGF/kg must be given, the dosage may be divided and injected subcutaneously at two or more sites.

If, in conjunction with standard gonadotropin therapy, the IGF/IGFBP-3 complex is administered to humans twice a week, each dose of complex is preferably about 0.05 to 10 mg IGF/kg of body weight, complexed to an equimolar amount of IGFBP-3. More preferably, for twice weekly administration, the dose of the complex is about 0.1 to 7.5 mg IGF/kg, complexed to an equimolar amount of IGFBP-3. Most preferably, for twice weekly administration, the dose of the complex is about 0.5 to 5 mg IGF/kg, complexed to an equimolar amount of IGFBP-3. There is no known upper limit of dosage; however, it is preferable that a single dose not exceed 10 mg IGF/kg of body weight, when the IGF is complexed to an equimolar amount of IGFBP-3. These doses of IGF/IGFBP-3 complex are not expected to cause significant hypoglycemia since the IGF-IGFBP-3 slowly releases IGF to cellular insulin receptors.

Preferably, the infertile patient is started with a relatively low dose of the complex, such as 0.05 mg IGF/kg of body weight/day in conjunction with standard gonadotropin therapy. The various factors given above should be monitored to determine if there is improvement. Preferably, the patient produces sufficient mature ovarian follicles or sufficient motile sperm following such treatment. If the patient improves with the low dose, the low dose preferably should be continued with standard gonadotropin therapy until a successful conception by normal means or by *in vitro* fertilization/embryo transfer is achieved. Such an outcome may require several rounds of therapy through several reproductive cycles.

If the patient does not respond to standard gonadotropin therapy plus low dose IGF/IGFBP-3 complex with sufficient production of mature oocytes or sperm, the dose of complex should be increased gradually until such an outcome is achieved.

The invention has been disclosed by direct description. Following are examples showing the efficacy of the IGF/IGFBP-3 complex in stimulating processes critical to the maturation of oocytes and sperm and in stimulating fertility. The examples are only examples and should not be taken in any way as limiting to the scope of the process.

### EXAMPLES

### Example 1

This experiment shows the effect of the rhIGF-I/IGFBP-3 complex alone and in conjunction with follicle stimulating hormone (FSH) on estradiol production in cultured ovarian granulosa cells isolated from patients with polycystic ovarian disease (PCOD). The level of estradiol, a crucial hormone in ovarian follicle development, is low in the fluid from ovarian follicles isolated from patients with PCOD. This is thought to cause the cessation in follicular development that characterizes PCOD.

Granulosa cells are prepared from PCOD patients' ovaries which have been removed during a total abdominal hysterectomy. Fluid from individual follicles is collected by aspiration. Then the follicles are dissected from the ovaries and cut open. From the open follicles, granulosa cells are scraped and washed free from the basal lamina. Cells from several follicles are pooled and cultured in serum-free McCoy's 5a medium in multi-well culture dishes for 2, 4, or 6 days. The estradiol precursor androstenedione is added at 10⁻⁷ M and the rhIGF-I/IGFBP-3 complex at doses in the range from 0 to 100 ng IGF-I/ml complexed to an equimolar amount of IGFBP-3. FSH in the range from 0 to 100 ng/ml is added to cultures with and without the rhIGF-I/IGFBP-3 complex. Estradiol secreted into the culture medium is assayed by a standard radioimmunoassay (RIA).

Untreated cultures produce detectable estradiol for only the first two-day culture period and not thereafter. When either the rhIGF-I/IGFBP-3 complex or FSH are added to the cultures on day 0, the cells respond with a dose-related increase in the production and secretion of estradiol into the culture medium. The effect of the combination of FSH and the IGF-I/IGFBP-3 complex on estradiol production is substantially greater than either factor alone. This effect is observed throughout the course of the culture period.

### Example 2

This experiment shows the effect of the rhIGF-I/IGFBP-3 complex on superovulation prior to *in vitro* fertilization/embryo transfer (IVF/ET) in "poor responder" patients. The patients in this study are PCOD patients who had a suboptimal response (ie. fewer than 6 oocytes collected and fewer than 4 embryos developed) with at least one IVF/ET cycle of ovarian stimulation using the standard combined regimen of gonadotropin releasing hormone (GnRH) agonist and human menopausal gonadotropin (hMG).

The patients are assigned to one of two groups: One group receives placebo and one group receives rhIGF-I/IGFBP-3 complex, in addition to standard gonadotropin treatment for IVF/ET. The placebo and rhIGF-I/IGFBP-3 complex (0.05 to 0.5 mg rhIGF-I/kg/day complexed to an equimolar amount of rhIGFBP-3) are administered by daily subcutaneous injection throughout the period of hMG treatment until hCG administration. The standard gonadotropin therapy given to both groups is as follows: At the beginning of the study, ovarian suppression, as measured by reduced plasma estradiol levels, is accomplished by GnRH agonist treatment. This treatment is maintained daily until hCG treatment. Following the establishment of ovarian suppression, hMG is administered at a rate of at least 3 ampules per day for at least 6 days. Increases in the daily dosage or days of treatment are determined by the follicular response of each subject as assessed by ultrasound scans of the patients' ovaries. hCG is administered when plasma estradiol levels are sufficiently elevated and at least three large follicles can be detected. Oocytes are recovered by transvaginal aspiration and subjected to the standard IVF/ET culture and fertilization procedures.

Treatment of these "poor-responding" PCOD patients with the rhIGF-I/IGFBP-3 complex results in a significant reduction in the number of ampules of gonadotropin required to stimulate superovulation compared to those treated with placebo. Furthermore, more follicles develop, and more oocytes are collected, fertilize and cleave in rhIGF-I/IGFBP-3 complex treated patients than in placebo-treated patients. These data support the utility of using the IGF/IGFBP-3 complex in the treatment of infertility.

### Example 3

This experiment demonstrates that the rhIGF-I/IGFBP-3 complex increases testicular growth and steroidogenic response in growth hormone deficient mice. Immature Snell dwarf mice, which are growth hormone deficient and exhibit delayed testicular maturation, are used in the study. The animals receive either placebo or rhIGF-I/IGFBP-3 complex (1.0 to 10 mg rhIGF-I/kg/day complexed to an equimolar amount of rhIGFBP-3) in daily subcutaneous injections for 7 to 14 days. At the end of the treatment period, an hCG injection is administered and blood and various organs are collected. Plasma testosterone and IGF-I levels are measured using standard RIA procedures and testicular hCG binding sites are measured by a radioreceptor assay.

Compared to placebo-treated animals, administration of the rhIGF-I/IGFBP-3 complex substantially increases circulating IGF-I and testicular weight in the Snell dwarf mice. Leydig cell function in rhIGF-I/IGFBP-3 complex treated mice is also stimulated compared to placebo treated animals, as evidenced by a significant increase in plasma testosterone levels following the hCG challenge, and in testicular hCG binding sites. These results demonstrate that the IGF/IGFBP-3 complex promotes testicular maturation.

### Example 4

This experiment shows that the rhIGF-I/IGFBP-3 complex promotes spermatogenesis in patients who have failed to respond to standard gonadotropin therapy alone. The patients included in the study are those diagnosed as suffering from hypogonadotropic hypogonadism on the basis of delayed puberty and reduced serum gonadotropin and testosterone levels. In addition, the included patients have failed to respond to standard gonadotropin therapy with improved levels of serum testosterone or sperm production.

Patients are treated with standard gonadotropin therapy which consists of 24 weeks of thrice weekly intramuscular injections of hMG, FSH and LH and twice weekly injections of hCG. This standard therapy is supplemented with daily subcutaneous injections of either placebo or rhIGF-I/IGFBP-3 complex (0.05 to 0.5 mg IGF-I/kg/day complexed to an equimolar amount of rhIGFBP-3) for the treatment period. Blood and semen samples are collected during weeks 12 and 24. Testicular volume is measured by ultrasonography at the end of the treatment period. Serum IGF-I and testosterone levels are measured by standard RIAs.

The patients receiving the rhIGF-I/IGFBP-3 complex in addition to standard gonadotropin therapy show a significant increase in serum IGF-I and testosterone concentrations compared to placebo-treated patients, especially at 24 weeks. Semen volume and sperm density also significantly increase in the rhIGF-I/IGFBP-3 complex treated group, although testicular volume does not increase in all complex-treated patients. Semen volume and sperm density increases have been associated with successful improvement in fertility and demonstrate the utility of combined gonadotropin and IGF/IGFBP-3 complex therapy in treating male infertility.

## Claims

1. The use of a complex comprising an insulin-like growth factor (IGF) and insulin-like growth factor binding protein-3 (IGFBP-3) for the manufacture of a medicament for alleviating or treating a reproductive disorder associated with a failure of gamete maturation.

2. The use according to claim 1 wherein the complex comprises equimolar amounts of IGF and IGFBP-3.

3. The use according to claim 1 or 2 wherein the IGF is IGF-I.

4. The use according to claim 3 wherein the IGF is recombinant human IGF-I.

5. The use according to claim 1 or 2 wherein the IGF is IGF-II.

6. The use according to claim 5 wherein the IGF-II is recombinant human IGF-II.

7. The use according to any preceding claim wherein the IGFBP-3 is recombinant human IGFBP-3.

8. The use according to any preceding claim wherein the medicament is adapted for parenteral administration.

9. The use according to claim 8 wherein the medicament is adapted for administration by subcutaneous injection.

10. The use according to any preceding claim wherein the amount of complex results in an increase in serum estrogen in females or serum androgen in males.

11. The use according to any preceding claim wherein medicament is adapted so that the amount of complex administered is at least about 0.05 mg IGF/kg of body weight/day.

12. The use according to any preceding claim wherein the medicament is to be administered to a mammal.

13. The use according to any preceding claim wherein the disorder is polycystic ovarian disease or anovulation.

14. The use according to any of claims 1 to 12 wherein the disorder can be treated with standard gonadotropin stimulation, superovulation, *in vitro* fertilization and embryo transfer therapies.

15. The use according to any of claims 1 to 12 wherein the disorder is poor response to standard gonadotropin stimulation of normal ovulation.

16. The use according to claim 15 wherein the disorder is male infertility.

17. The use according to claim 16 wherein the disorder is hypogonadotropic hypogonadism.

18. The use according to any of claims 1 to 12 wherein the disorder is also associated with failure to respond to standard gonadotropin therapy.

## Patentansprüche

1. Verwendung eines Komplexes, der einen insulinähnlichen Wachstumsfaktor (IGF) und das insulinähnlicher Wachstumsfaktor-Bindungsprotein-3 (IGFBP-3) umfasst, für die Herstellung eines Medikaments zur Linderung oder Behandlung einer Störung der Reproduktionsfähigkeit die mit einem Ausbleiben der Gametenreifung verbunden ist.

2. Verwendung nach Anspruch 1, wobei der Komplex äquimolare Mengen von IGF und IGFBP-3 umfasst.

3. Verwendung nach Anspruch 1 oder 2, wobei der IGF IGB-I ist.

4. Verwendung nach Anspruch 3, wobei der IGF rekombinanter menschlicher IGF-I ist.

5. Verwendung nach Anspruch 1 oder 2, wobei der IGF IGF-II ist.

6. Verwendung nach Anspruch 5, wobei der IGF-II rekombinanter menschlicher IGF-II ist.

7. Verwendung nach einem der vorangehenden Ansprüche, wobei der IGFBP-3 rekombinanter menschlicher IGFBP-3 ist.

8. Verwendung nach einem der vorangehenden Ansprüche, wobei das Medikament für eine parenterale Verabreichung angepaßt ist.

9. Verwendung nach Anspruch 8, wobei das Medikament für eine Verabreichung durch subkutane Injektion angepaßt ist.

10. Verwendung nach einem der vorangehenden Ansprüche, wobei die Menge des Komplexes zu einem Anstieg des Serum-Östrogens in Frauen und des Serum-Androgens in Männern führt.

11. Verwendung nach einem der vorangehenden Ansprüche, wobei das Medikament so angepaßt ist, daß die Menge des verabreichten Komplexes mindestens etwa 0,05 mg IGF/kg Körpergewicht/Tag beträgt.

12. Verwendung nach einem der vorangehenden Ansprüche, wobei das Medikament an einen Säuger verabreicht werden soll.

13. Verwendung nach einem der vorangehenden Ansprüche, wobei die Störung eine polycystische Eierstockerkrankung oder eine Anovulation ist.

14. Verwendung nach einem der Ansprüche 1 bis 12, wobei die Störung durch Standard-Gonadotropinstimulation, Superovulation, in-vitro- Fertilisation und Embryotransfertherapien behandelt werden kann.

15. Verwendung nach einem der Ansprüche 1 bis 12, wobei die Störung eine schwache Antwort auf Standard-Gonadotropinstimulation bei normaler Ovulation ist.

16. Verwendung nach Anspruch 15, wobei die Störung männliche Infertilität ist.

17. Verwendung nach Anspruch 16, wobei die Störung hypogonadotroper Hypogonadismus ist.

18. Verwendung nach einem der Ansprüche 1 bis 12, wobei die Störung auch mit einer mangelnden Antwort auf Standard-Gonadotropintherapie verbunden ist.

## Revendications

1. Utilisation d'un complexe comprenant un facteur de croissance analogue à l'insuline (IGF, insulin-like growth factor ; somatomédine) et la protéine-3 de liaison au facteur de croissance analogue à l'insuline (IGFBP-3, insulin-like growth factor binding protein-3) pour la préparation d'un médicament destiné à atténuer ou traiter un dysfonctionnement de la reproduction associé à une défaillance de la maturation des gamètes.

2. Utilisation selon la revendication 1, dans laquelle le complexe comprend des quantités équimolaires d'IGF et d'IGFBP-3.

3. Utilisation selon la revendication 1 ou 2, dans laquelle l'IGF est l'IGF-I.

4. Utilisation selon la revendication 3, dans laquelle l'IGF est l'IGF-I recombinant humain.

5. Utilisation selon la revendication 1 ou 2, dans laquelle l'IGF est l'IGF-II.

6. Utilisation selon la revendication 5, dans laquelle l'IGF-II est l'IGF-II recombinant humain.

7. Utilisation selon l'une quelconque des revendications précédentes, dans laquelle l'IGFBP-3 est l'IGFBP-3 recombinant humain.

8. Utilisation selon l'une quelconque des revendications précédentes, dans laquelle le médicament est adapté à une administration parentérale.

9. Utilisation selon la revendication 8, dans laquelle le médicament est adapté à une administration par injection sous-cutanée.

10. Utilisation selon l'une quelconque des revendications précédentes, dans laquelle la quantité de complexe provoque une augmentation d'oestrogène sérique chez les femelles ou d'androgène sérique chez les mâles.

11. Utilisation selon l'une quelconque des revendications précédentes, dans laquelle le médicament est adapté pour que la quantité de complexe administrée soit d'au moins environ 0,05 mg d'IGF/kg de masse corporelle/jour.

12. Utilisation selon l'une quelconque des revendications précédentes, dans laquelle le médicament doit être administré à un mammifère.

13. Utilisation selon l'une quelconque des revendications précédentes, dans laquelle le dysfonctionnement est une maladie ovarienne polykystique ou une anovulation.

14. Utilisation selon l'une quelconque des revendications 1 à 12, dans laquelle le dysfonctionnement peut être traité par des traitements standard de stimulation par une gonadotrophine, de superovulation, de fécondation *in vitro* et de transfert d'embryon.

15. Utilisation selon l'une quelconque des revendications 1 à 12, dans laquelle le dysfonctionnement est une réponse faible à la stimulation standard par une gonadotrophine de l'ovulation normale.

16. Utilisation selon la revendication 15, dans laquelle le dysfonctionnement est une stérilité mâle.

17. Utilisation selon la revendication 16, dans laquelle le dysfonctionnement est un hypogonadisme hypogonadotrophique.

18. Utilisation selon l'une quelconque des revendications 1 à 12, dans laquelle le dysfonctionnement est également associé à une défaillance de réponse à un traitement par gonadotrophine standard.
